# EUROPEAN PATENT SPECIFICATION

(11) **EP 2 018 198 B1**
(45) Date of publication and mention of the grant of the patent: **08.05.2013**
(21) Application number: 06761011.3
(22) Date of filing: 24.08.2006
(51) Int. Cl.: A61M 5/50, A61M 5/315

(54) **SINGLE USE SYRINGE**
SPRITZE FÜR DEN EINMALGEBRAUCH
SERINGUE À USAGE UNIQUE

(30) Priority: 17.05.2006 AU 2006902628 P
(43) Date of publication of application: 28.01.2009
(73) Proprietor: Morgan Meditech Inc., Kowloon Hong Kong (CN)
(72) Inventor: WALSH, Allan, Medowie, NSW 2318 (AU); TAN, Feiyan, Guangdong 528305 (CN)
(74) Representative: Maiwald, Walter
(86) International application number: PCT/AU2006/001228
(87) International publication number: WO 2007/131259

(56) References cited:
- EP-A1- 0 409 134
- WO-A1-88/10127
- US-A- 4 775 364
- US-A- 4 932 941
- US-A- 5 415 638
- US-A1- 2004 176 722
- US-A1- 2005 240 149

## Description

### Field of the Invention

The present invention relates to a single use syringe. In particular, the present invention relates to a self-destructing syringe suitable for single delivery of medication and vaccinations.

### Background of the Invention

The risk of disease transmission resulting from the re-use of syringes is a serious problem around the world, and in particular in developing and third world countries. Diseases such as HIV (Human immunodeficiency virus) and Hepatitis B along with other blood borne diseases are readily transmitted between persons when a syringe is reused multiple times. Such problems are particularly prevalent among intravenous drug users who commonly share the same syringe without adequate sterilisation between users.

In addition, in some developing countries which lack suitably developed health standards, syringes used for medical purposes such as immunisations are poorly sterilised and subsequently re-used. This is known to sometimes result in the transmission of blood bom diseases between patients when inadequate methods of sterilisation are practiced.

Single-use syringes are known. However, a problem with such syringes is that they are typically significantly more expensive to manufacture than conventional syringes which permit repeated use. Accordingly, in applications such as large-scale immunisations, the significant additional expense associated with using existing single-use syringes is not generally feasible in developing or third world countries.

US 2005/240149 A1 discloses a self-destructive syringe. Upon completion of the injection, the locking member is left in the barrel, and the plunger is pulled out of the barrel to prevent reuse of the syringe.

US 5,415,638 A describes a safety syringe with an interchangeable and retractable needle platform.

In US 4,932,941 A, a non-reusable syringe is disclosed, which has its piston locked in its forward dispense position after it is used.

EP 0 409 134 A1 discloses a non-reusable syringe, which contains two retention steps to retain the piston once utilized.

### Object of the Invention

It is the object of the present invention to overcome or substantially ameliorate at least one of the above disadvantages, or at least to provide a useful alternative.

### Summary of the Invention

The present invention provides a single use syringe comprising:
a hollow barrel having a longitudinal axis extending in a longitudinal direction and including a first engagement formation formed on an internal wall of said barrel;
a needle tip fixed relative to said barrel, said needle tip being in fluid communication with said barrel; and

The stem and head are preferably connected by a frangible region of the plunger.

The frangible region preferably includes a clevis shaped projection connected to the stem and a pin connected to the head.

The frangible region preferably includes four lugs which frangibly connect the pin to the clevis shaped portion.

The first engagement formation preferably includes one or more teeth formed on an internal wall of the barrel, and the second engagement formation includes a shoulder formed on the head and being engageable with the one or more teeth.

The predetermined longitudinal force required to separate the stem from the head is preferably between about 5N and 15N.
a plunger insertable within said barrel, said plunger having a stem and a head separably connected to said stem, said head including a second engagement formation located at a leading end of said head;
said plunger being displaceable through a longitudinal stroke in said longitudinal direction to a captive position at an end of said stroke in which said first engagement formation engages said second engagement formation, thereby securing the plunger within the barrel,
wherein in the captive position, the head is secured thereby preventing movement of the head through said stroke, and said stem is adapted to separate from the head if an attempt is made to longitudinally displace the stem in either an insertion or retraction direction of the stroke, by application of a predetermined longitudinal force, such that said head remains secured within said barrel, characterized by a detent arrangement associated with said barrel and said plunger, wherein said detent arrangement is configured to inhibit displacement of said plunger in said longitudinal direction as said plunger approaches said captive position, and
wherein said detent arrangement includes a first detent portion formed on said internal wall of said barrel and a second detent portion formed on said stem, said first detent portion being adapted to engage said second detent portion as said plunger approaches said captive position.

The first detent portion preferably includes a projection that projects from the internal wall of the barrel into a hollow of the barrel, thereby locally reducing a transverse cross section of the hollow.

The second detent portion preferably includes a protuberance formed on the stem.

The protuberance is preferably deformable upon engagement with the projection.

The protuberance preferably includes two ribs protruding from opposing sides of the stem.

Each rib preferably includes a central portion connected to the stem at first and second ends, such that an aperture is defined between the central portion and the stem.

The needle mount preferably includes a radially extending lip which is captively received within a corresponding annular groove formed within the leading hollow portion.

In a further aspect, the present invention provides a method of forming a barrel of a syringe, said method including the steps of:
locating a blank of plastic within a die having a longitudinal axis extending in a longitudinal direction, and
pressing said blank from a first side sides along said longitudinal axis, thereby forming a leading hollow portion for receipt of a needle mount,
pressing said blank from a second opposing side thereby forming a trailing hollow portion for receipt of a plunger,
wherein said pressing from said first side and said pressing from said second side also forms a first engagement formation for engaging a leading portion of a lead of the plunger, said first engagement formation being defined by a restriction which separates said leading hollow portion and said trailing hollow portion;
characterized in that a first detent portion is formed on the internal wall of said barrel which is configured to be part of a detent arrangement associated with said barrel and said plunger, wherein said detent arrangement is configured to inhibit displacement of said plunger in said longitudinal direction as said plunger approaches a captive position, and in that said first detent portion is adapted to engage a second detent portion formed on the stem of said plunger when said plunger approaches said captive position.

The step of forming the first engagement formation preferably includes forming one or more moulded teeth.

### Brief Description of the Drawings

Preferred embodiments of the present invention will be described by way of example only, with reference to the accompanying drawings, in which;
Fig. 1 is a sectional side view of a single use syringe;
Fig. 2 is a cross-sectional view of the plunger of the syringe of Fig. 1;
Fig. 3 is a detailed cross-sectional view of the trailing end of the plunger of Fig. 2;
Fig. 4 is a detailed view of the leading end of the plunger of Figs. 2 to 3;
Fig. 5 is a leading end view of the barrel of the syringe of in Fig. 1;
Fig. 6 is a cross-sectional side view of the leading end of the barrel of the syringe of Fig. 1;
Fig. 7 is a cross-sectional side view of the syringe of Fig. 1 after completion of an injecting operation;
Fig. 8 is a cross-sectional side view of a needle mount of the syringe of Fig. 1;
Fig 9 is a leading end view of the needle mount of Fig. 8;
Fig. 10 is a cross-sectional side view of a rubber piston seal of the syringe of Fig. 1;

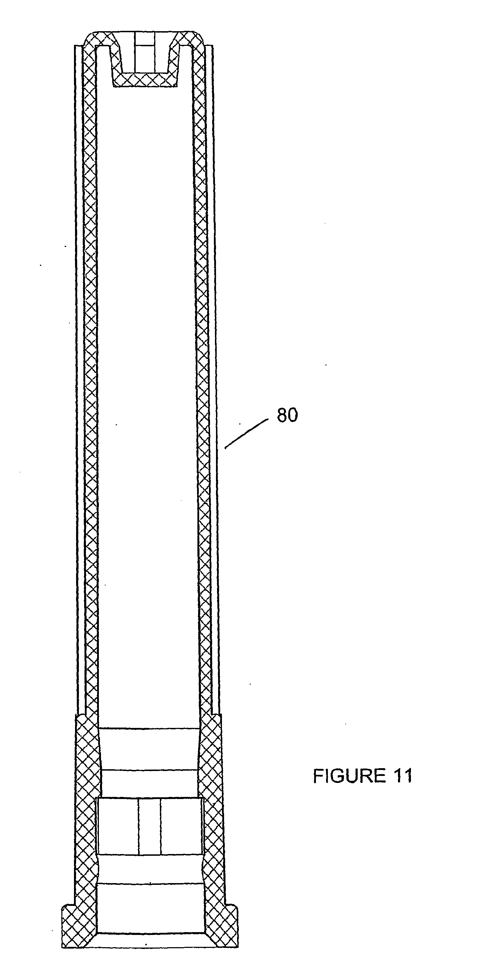

Fig. 11 is a cross-sectional side view of a needle cap for covering the needle of the syringe of Fig. 1; and

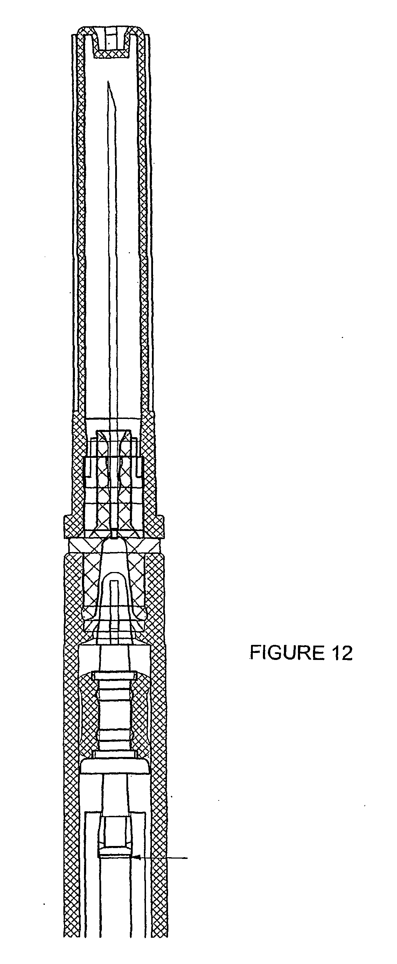

Fig. 12 is a cross-sectional side view of the leading end of the syringe of Fig. 1 with the needle cap.

### Detailed description of the preferred embodiments

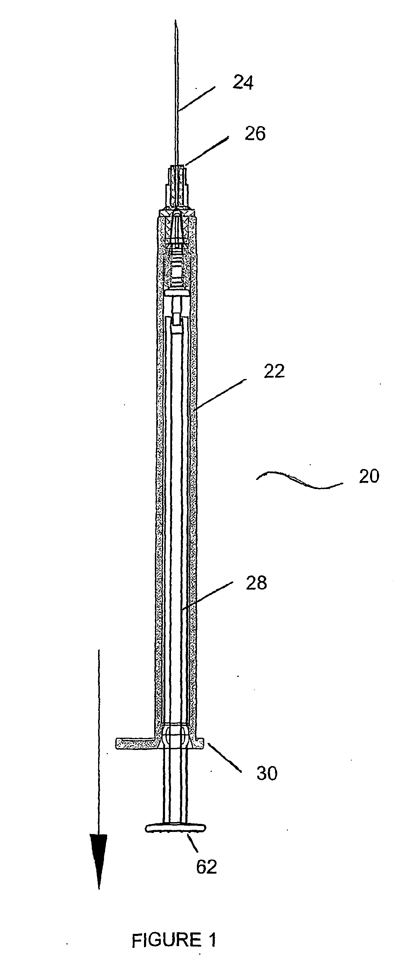

A single use syringe is depicted in the drawings and indicated by the reference numeral 20. As seen in Fig. 1, the syringe 20 includes a hollow barrel 22 having a longitudinal axis X, and the barrel 22 is connected to a stainless steel needle tip 24, located at the leading end 26 of the barrel 22. The barrel 22 is made from a clear plastic material, enabling the volume of fluid contained therein to be externally seen. The syringe 20 also includes a plunger 28 which is insertable into an opening formed in the trailing end 30 of the barrel 22.

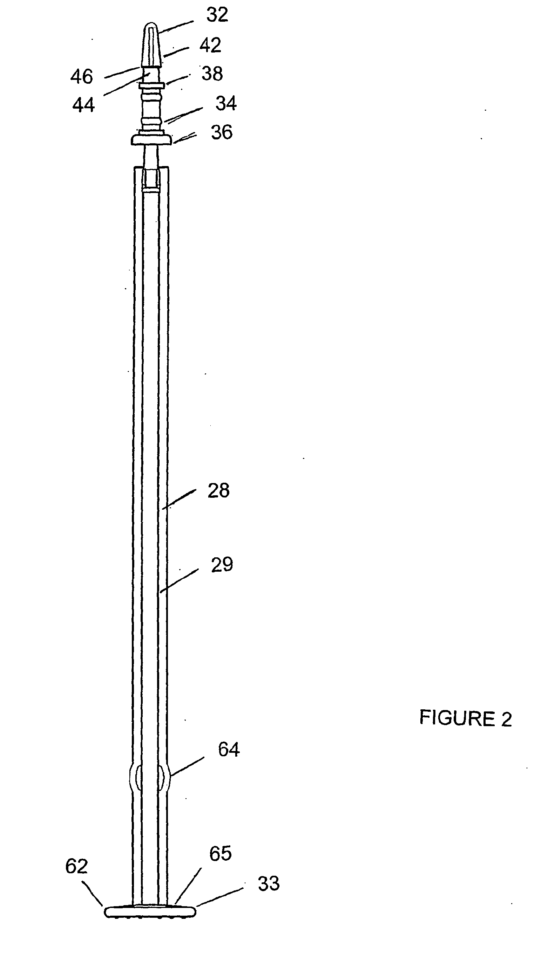

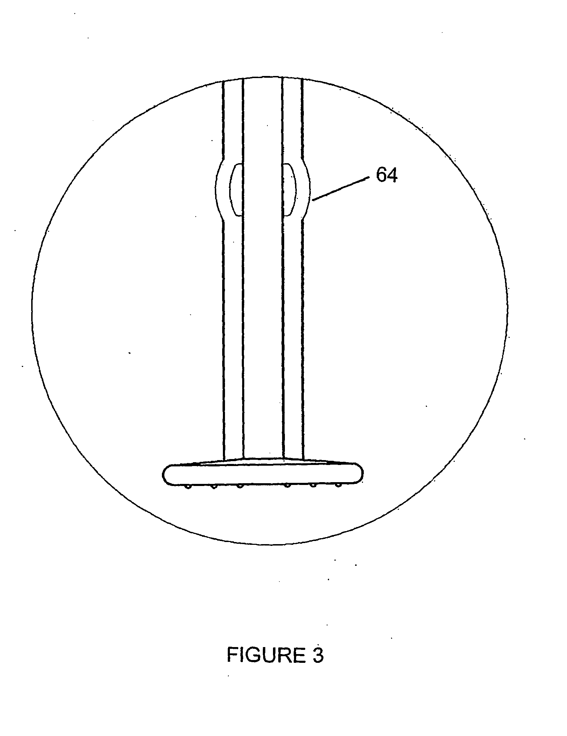

Figs. 2 to 4 show the plunger 28 in isolation. The plunger 28 includes a head 34 which is insertable into the trailing hollow end 30 of the barrel 22. The plunger 28 also includes a stem 29 separably connected to the head 34. The head 34 and the stem 29 are connected by a frangible region 48 of the plunger 28, and the plunger 28 is displaceable through a longitudinal stroke in the longitudinal direction, to a captive position towards an end of the stroke. The captive position is typically reached when the head 34 has travelled through at least 95% of the stroke, and in the captive position, the head 34 is secured thereby preventing movement of the head 34 through said stroke in either an insertion or retraction direction.

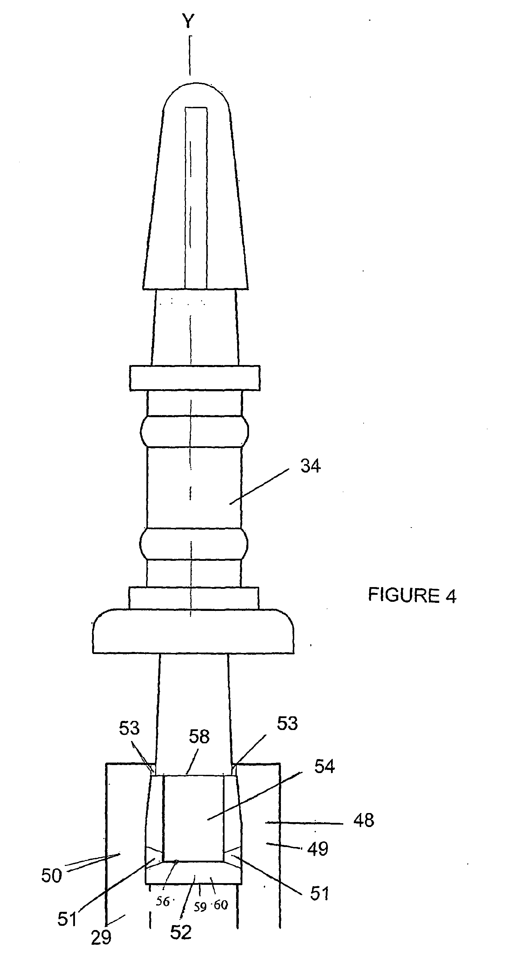

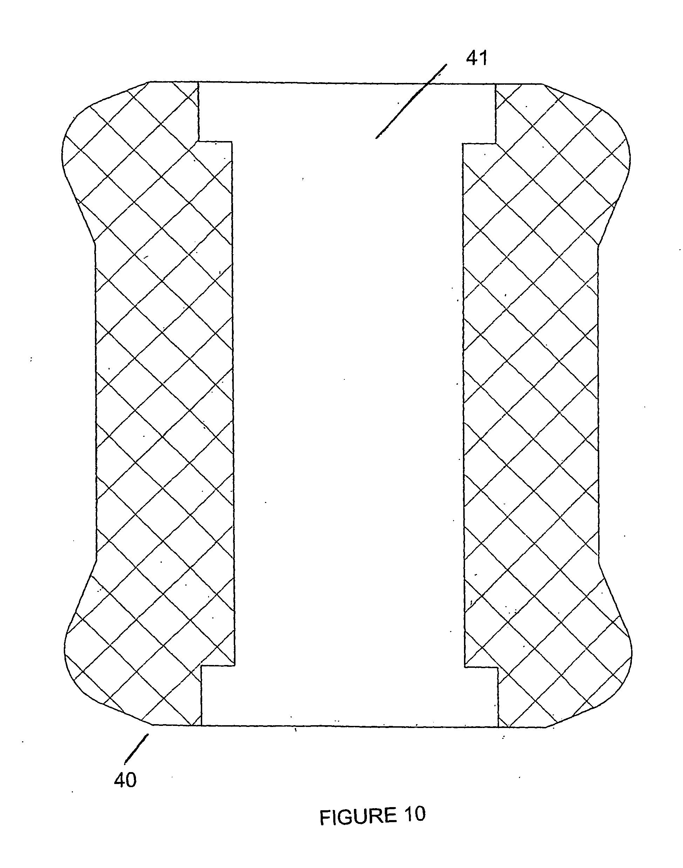

As seen in Figs. 2 and 4, the head 34 has two annularly projecting shoulders 36, 38 which act as a support to retain a flexible piston seal 40 which is shown in Fig. 10.

The piston seal 40 is manufactured from a synthetic rubber and has raised annular rings on its outer surface, which interferingly contact the inner wall of the barrel 22, to form a liquid tight seal. A hole 41 is formed through the piston seal 40, for seating the piston seal 40 on the head 34, between the shoulders 36, 38.

The head 34 terminates at a tapered projection 42 with a rounded tip positionable at the needle tip 24 end of the syringe 20, such that the diameter of the tapered projection 42 increases as it progresses towards the stem 29, and terminates at a second engagement formation 46 in the form of an annular shoulder 46. The projection 42 is connected to a neck 44 of the head 34 having a smaller diameter than the shoulder 46, such that the annular shoulder 46 extends radially beyond the neck 44.

The frangible region 48 of the plunger 28 is best seen in Fig. 4. The frangible region 48 preferably includes a clevis shaped formation 49 connected to the stem 29, which is formed by two opposing arms 50 which each extend parallel to the longitudinal axis Y of the plunger 28. The arms 50 are separated by a space 52, through which the axis Y passes. The frangible region 48 also includes a pin 54 connected to the head 34 and projecting into the space 52 between the opposing arms 50, such that the pin 54 is coaxial with the axis Y.

The end 56 of the pin 54 is attached on two sides to the arms 50 by two frangible lugs 51. The pin 54 has a region 58 in which the diameter steps up to an increased diameter, which commences near the ends of the arms 50. At the region 58 of increased diameter, the pin 54 is also connected to the end portion of the arms 50, by two further frangible lugs 53. Accordingly, the head 34 is connected to the stem 29 at four locations, which are the frangible lugs 51, 53. The dimensions of the frangible lugs 53 are preferably 0.4mm by 0.3mm, and the dimensions of the frangible lugs 51 are preferably 0.0175mm by 0.3mm.

While the stem 29 is connected to the head 34, a gap 60 is present between the base 59 of the clevis shaped formation 49 and the end 56 of the pin 54.

The trailing end of the plunger 28 has a finger pad 62 formed therein, which provides a surface that a user can press to drive the plunger 28 into the barrel 22.

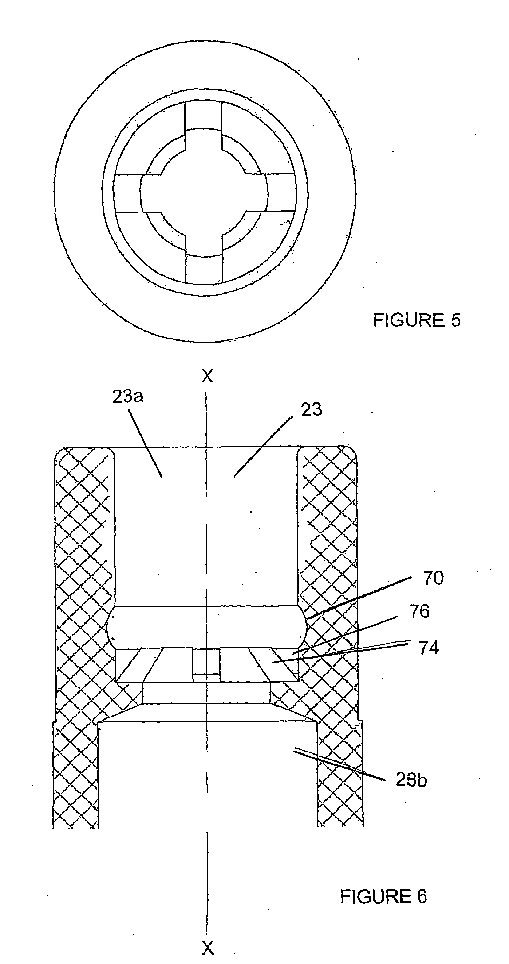

Fig. 6 shows the barrel 22, which is formed by a plastic moulding operation, in which it is pressed or stamped in a die, such that forces are applied to the leading end 26, along the longitudinal axis X of the barrel 22 such that a leading hollow 23a is formed in the blank. A force is also applied in an opposite direction along the longitudinal axis X of the barrel 22 such that a trailing hollow 23b is also formed in the blank between the leading and trailing ends 26, 30 of the barrel 11.

The pressing operation also forms a first engagement portion defined by a restriction which separates the leading hollow portion 23a and the trailing hollow portion 23b. The step of forming the first engagement formation preferably includes forming one or more moulded teeth. An advantage of the forming process, is that it permits the teeth to be formed during the same process as the forming of the leading and trailing hollow portions 23a, 23b.

A detent arrangement is associated with the barrel 22 and the plunger 28 in the form of a first detent portion 71 formed on an inner wall of the barrel 22 in the form of a projection 71, and a corresponding second detent 64 portion in the form of a protuberance 64 on said stem 28. The projection 71 is engageable with the protuberance 64, such that the detent arrangement is configured to inhibit displacement of the plunger 28 in the longitudinal direction as the plunger approaches the captive position.

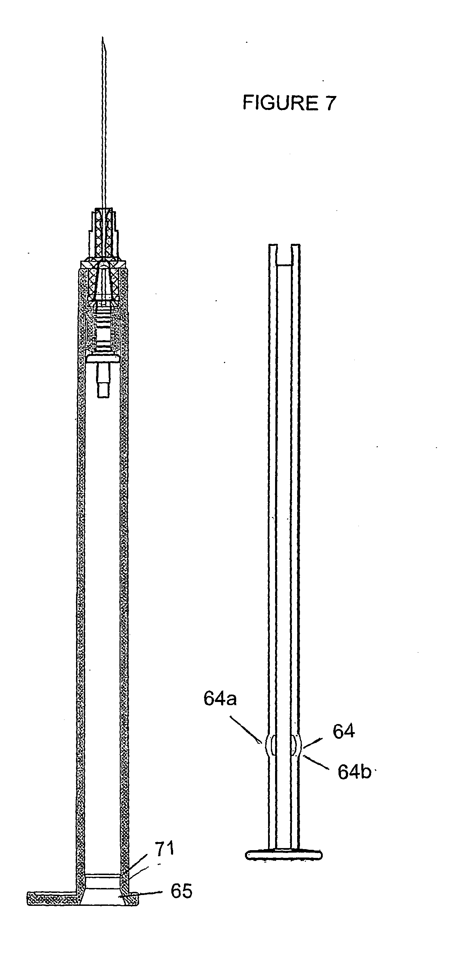

As shown in Fig. 7, the initial opening of the barrel 22 at the trailing end 30 has a tapered region 65, such that the entrance to the trailing hollow portion 23b is sufficiently wide to permit a user to easily insert the head 34 and the rubber piston seal 40 into the trailing hollow portion 23b. The tapered region 65 is adjacent to the first detent portion 71 which is embodied in the form of a projection 71 that projects from the internal wall of the barrel 22 into the hollow within the barrel 22 thereby locally reducing the transverse cross section of the hollow. The protuberance 64 preferably includes two ribs 64a, 64b protruding from opposing sides of the stem 29. Each rib 64a, 64b includes a central portion connected to the stem 29 by first and second ends, such that an aperture is defined between the central portion and the stem 29.

Adjacent to the projection 71 towards the leading end 26, the transverse cross sectional area of the trailing hollow portion 23b increases, such that the projection 71 acts as a restriction, locally reducing the transverse cross-sectional area of the trailing hollow portion 23b.

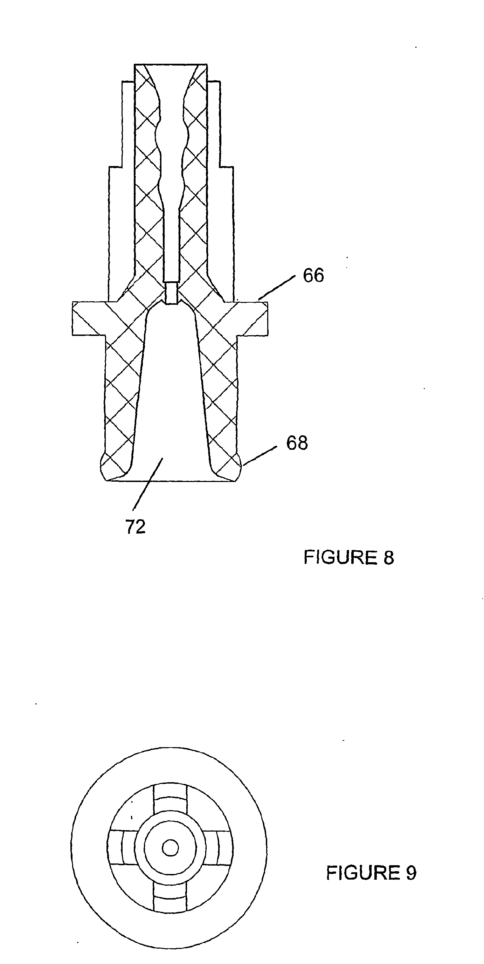

The needle 24 is permanently mounted to a needle mount 66 in the form of a needle holding bush 66 which is best seen in Fig. 8. The needle mount 66 has a radially extending lip 68 which engages with a corresponding annular groove 70 within the barrel 22, and best seen in Fig. 6. When the annular lip 68 of the needle mount 66 is snapped into the annular groove 70, the needle 24 permanently connects with the syringe 20. A generally cone shaped hollow 72 with a rounded end is formed in the trailing end of the needle mount 66.

Referring to Figs 5 and 6, the first engagement formation 74 is in the form of four teeth 74 formed within the barrel 22 toward the leading end 26, and adjacent to and trailing the annular groove 70. In an alternative arrangement not shown in the drawings, there may be more or less than four teeth 74. The teeth 74 project diagonally inwardly within the hollow barrel 22, such that the teeth 74 proj ect toward the leading end 26 of the barrel 22.

As seen in Fig. 6, there is a space 76 formed between each tooth 74 and the wall of the barrel 24. The teeth 74 are elastically deformable, and the teeth 74 are preferably spaced evenly around the circumference of the barrel 22.

The syringe 20 includes a needle cap 80 which covers the tip of the needle 34, and engages with the needle mount 66 by a snap lock.

The operation of the syringe 20 will now be described. When an injection of liquid in the form of medication or an immunisation is to be administered, a medical practitioner removes the needle cap 80 which exposes the needle tip 24. The practitioner then applies a force with his/her thumb to the finger pad 62, to drive the piston 34 into the barrel 22. As the piston 34 gets close to the end of its stroke such that the head 34 is approaching the leading end 26 of the barrel 22, the protuberance 64 of the stem 29 enters into the tapered region 65, and subsequently engages the projection 71 of the barrel 22.

At this point of the insertion process, the protuberance 64 is in contact with and engages the projection 71, which inhibits movement of the plunger 28 in the insertion direction, thereby increasing the force required to insert the plunger 28 any further in the longitudinal direction. The user can feel an increased drag force, and is hence aware without visually looking at the syringe 20 that the head 34 is nearing the designated "fill" position.

Inserting the head 34 any further within the barrel 22 at this point will cause the barrel 22 to captively engage the head 34, thereby preventing the syringe 20 from being used.

The user then inserts the needle 24 into a vial of liquid (not shown) and subsequently retracts the stem 29 away from the needle 24 end of the syringe 20, to draw a quantity of the liquid into the barrel 22. The drawing operation is conducted when the user places the first and second fingers of one hand under two flanges 65 formed on the underside of the finger pad 62, located on either side of the stem 29. Markings on the side of the barrel 22 indicate the volume of the liquid contained therein, such that a desired dosage of liquid can be measured. The rubber piston seal 40 prevents leakage between the piston 34 and the inside wall of the hollow barrel 22.

The user then holds the syringe with the needle facing upwardly and applies a further force with his/her thumb to the finger pad 62, to again drive the piston 34 into the barrel 22. Any air present in the barrel 22 is then expelled from the syringe 20, leaving liquid only within the barrel 22.

The needle 24 is then inserted into a patient's muscle tissue or a vein as required, and the contents of the barrel 22 are injected into the patient by applying a further force to the finger pad 62. As the head 34 approaches the end of its stroke, the cone shaped projection 42 enters into the corresponding cone shaped hollow 72 within the needle mount 66. The shoulder 46 then comes into contact with the teeth 74, and as a further force is applied, the teeth 74 are elastically displaced radially towards the inner wall of the barrel 22. The shoulder 46 then passes the end of the teeth 74, and the teeth 74 spring back to their original position, restraining the shoulder 46 against being retracted from the barrel 22. At this stage, the injection is complete and the cone shaped head 42 is captively seated within the corresponding cone shaped hollow 72, such that substantially all of the liquid has been ejected from the barrel 22.

At this stage if a user makes an attempt to longitudinally displace the head stem 29 by applying a force to the stem 29 in either the insertion or retraction directions by application of a predetermined longitudinal force, the stem 29 separates from the head 34 by rupture of the four frangible lugs 51, 53, such that the head 34 remains secured within the barrel 22. The force required to rupture the four frangible lugs 51, 53 is typically in the range of 5N to 15N. However, this may be set to a desired level by altering the cross sectional area of the frangible lugs 51, 53.

Fig. 8 shows the syringe 20 after completion of the injecting procedure, in which the head 34 has separate from the stem 29. In the event that the stem 29 and head 34 are not separated upon the completion of the injection cycle, the separation will occur if an attempt is subsequently made to retract the head 34 from the barrel 22, or further insert the head 34 into the barrel 22.

When the injection is complete, the complete syringe 20 is disposed of in a sharps container.

## Claims

1. A single use syringe (20) comprising:
a hollow barrel (22) having a longitudinal axis extending in a longitudinal direction and including a first engagement formation (74) formed on an internal wall of said barrel (22);
a needle tip (24) fixed relative to said barrel (22), said needle tip (24) being in fluid communication with said barrel (22); and
a plunger (28) insertable within said barrel (22), said plunger (28) having a stem (29) and a head (34) separably connected to said stem (29), said head (34) including a second engagement formation (46) located at a leading end of said head (34);
said plunger (28) being displaceable through a longitudinal stroke in said longitudinal direction to a captive position at an end of said stroke in which said first engagement formation (74) engages said second engagement formation (46), thereby securing the plunger (28) within the barrel (22),
wherein in the captive position, the head (34) is secured thereby preventing movement of the head (34) through said stroke, and said stem (29) is adapted to separate from the head (34) if an attempt is made to longitudinally displace the stem (29) in either an insertion or retraction direction of the stroke, by application of a predetermined longitudinal force, such that said head (34) remains secured within said barrel (22),
**characterized by** a detent arrangement associated with said barrel (22) and said plunger (28), wherein said detent arrangement is configured to inhibit displacement of said plunger (28) in said longitudinal direction as said plunger (28) approaches said captive position and,
wherein said detent arrangement includes a first detent portion (71) formed on said internal wall of said barrel (22) and a second detent portion (64) formed on said stem (29), said first detent portion (71) being adapted to engage said second detent portion (64) as said plunger (28) approaches said captive position.

2. The syringe (20) of claim 1, wherein the stem (29) and head (34) are connected by a frangible region (48) of said plunger (28).

3. The syringe (20) of claim 2, wherein the frangible region (48) includes a clevis shaped projection (49) connected to said stem (29) and a pin (54) connected to said head (34).

4. The syringe (20) of claim 3, wherein said frangible region (48) includes four lugs (51, 53) which frangibly connect the pin (54) to said clevis shaped portion (49).

5. The syringe (20) of any one of the preceding claims, wherein the first engagement formation (74) includes one or more teeth formed on an internal wall of said barrel (22), and said second engagement formation (46) includes a shoulder formed on said head and being engageable with said one or more teeth.

6. The syringe (20) of any one of the preceding claims, wherein said head (34) includes a piston seal (40) adapted to sealingly engage an inner wall of said barrel (22), wherein said second engagement formation (46) is located at a leading side of said piston seal (40).

7. The syringe (20) of any one of the preceding claims, wherein said predetermined longitudinal force required to separate the stem (29) from the head (34) is between about 5N and 15N.

8. The syringe (20) of any one of the preceding claims, wherein said captive position is reached when said head (34) has travelled through at least 95% of said stroke.

9. The syringe (20) of claim 1, wherein said first detent portion (71) includes a projection that projects from said internal wall of said barrel (22) into a hollow (23) of said barrel (22), thereby locally reducing a transverse cross section of said hollow (23).

10. The syringe (20) of claim 9, wherein said second detent portion (64) includes a protuberance formed on said stem (29).

11. The syringe (20) of claim 10, wherein said protuberance is deformable upon engagement with said projection.

12. The syringe (20) of claim 10 or 11, wherein said protuberance includes two ribs (64a, 64b) protruding from opposing sides of said stem (29).

13. The syringe (20) of claim 12, wherein each rib (64a, 64b) includes a central portion connected to the stem (29) at first and second ends, such that an aperture is defined between the central portion and said stem (29).

14. The syringe (20) of any one of the preceding claims, wherein said first engagement formation is integrally formed with said barrel (22).

15. The syringe (20) of any one of the preceding claims,
wherein said barrel (22) has a hollow (23) extending between a leading end (26) of said barrel (22) and a trailing end (30) of said barrel (22), said hollow (23) comprising a leading hollow portion (23a) and a trailing hollow portion (23b) separated by a restriction defined by said first engagement formation (74), and wherein said single use syringe (20) further comprises
a needle mount (66) mounted within said leading hollow portion (23a), wherein the needle tip (24) is mounted on said needle mount (66), and wherein the plunger (28) is insertable within said trailing hollow portion (23b), said plunger (28) being displaceable through a longitudinal stroke in said longitudinal direction to a captive position at an end of said stroke in which said first engagement formation (74) engages said second engagement formation (46), thereby securing the plunger (28) within the trailing hollow portion (23b).

16. The syringe (20) of claim 15, wherein said needle mount (66) includes a radially extending lip (68) which is captively received within a corresponding annular groove (70) formed within said leading hollow portion (23a).

17. A method of forming a barrel (22) of a syringe (20), said method including the steps of:
locating a blank of plastic within a die having a longitudinal axis extending in a longitudinal direction, and
pressing said blank from a first side along said longitudinal axis, thereby forming a leading hollow portion (23a) for receipt of a needle mount (66),
pressing said blank from a second opposing side thereby forming a trailing hollow portion (23b) for receipt of a plunger (28),
wherein said pressing from said first side and said pressing from said second side also forms a first engagement formation (74) for engaging a leading portion of a head (34) of the plunger (28), said first engagement formation (74) being defined by a restriction which separates said leading hollow portion (23a) and said trailing hollow portion (23b);
**characterized in that** a first detent portion (71) is formed on the internal wall of said barrel (22) which is configured to be part of a detent arrangement associated with said barrel (22) and said plunger (28), wherein said detent arrangement is configured to inhibit displacement of said plunger (28) in said longitudinal direction as said plunger (28) approaches a captive position, and **in that** said first detent portion (71) is adapted to engage a second detent portion (64) formed on the stem (29) of said plunger (28) when said plunger (28) approaches said captive position.

18. The method of claim 17, wherein said step of forming said first engagement formation (74) includes forming one or more moulded teeth.

## Patentansprüche

1. Einwegspritze (20) umfassend:
einen hohlen Zylinder (22) mit einer Längsachse, die sich in eine Längsrichtung erstreckt, und mit einer ersten Verbindungsstruktur (74), die auf einer inneren Wand des Zylinders (22) ausgeformt ist;
eine Nadelspitze (24), fixiert relativ zum Zylinder (22), wobei die Nadelspitze (24) in Fluidverbindung mit dem Zylinder (22) steht; und
einen Kolben (28), der in den Zylinder (22) eingeführt werden kann, wobei der Kolben (28) einen Hals (29) hat und einen Kopf (34), der mit dem Hals (29) trennbar verbunden ist, wobei der Kopf (34) eine zweite Verbindungsstruktur (46) hat, die am vorderen Ende des Kopfes (34) positioniert ist;
wobei der Kolben (28) durch einen Längshub in die Längsrichtung bis zu einer Fangposition an einem Ende des Hubs verschiebbar ist, in welcher sich die erste Verbindungsstruktur (74) mit der zweiten Verbindungsstruktur (46) verbindet und dadurch den Kolben (28) im Zylinder (22) sichert,
wobei der Kopf (34) in der Fangposition gesichert wird und dadurch eine Bewegung des Kopfes (34) durch den Hub verhindert wird, und der Hals (29) so gestaltet ist, dass er sich vom Kopf (34) trennt, falls versucht wird, den Hals (29) entweder in Einschubs- oder in Rückzugsrichtung des Hubes durch Aufwendung einer bestimmten in Längsrichtung gerichteten Kraft in Längsrichtung zu verschieben, so dass der Kopf (34) innerhalb des Zylinders (22) gesichert bleibt,
**dadurch gekennzeichnet, dass** eine Rasteinrichtung mit dem Zylinder (22) und mit dem Kolben (28) verbunden ist, wobei die Rasteinrichtung so gestaltet ist, dass sie die Verschiebung des Kolbens (28) in der Längsrichtung verhindert, sobald sich der Kolben (28) der Fangposition nähert, und
wobei die Rasteinrichtung ein erstes Rastteil (71), das auf der inneren Wand des Zylinders (22) ausgeformt ist, und ein zweites Rastteil (64), das auf dem Hals (29) ausgeformt ist, einschließt, wobei das erste Rastteil (71) so angepasst ist, dass es sich mit dem zweiten Rastteil (64) verbindet, sobald sich der Kolben (28) der Fangposition nähert.

2. Die Spritze (20) gemäß Anspruch 1, wobei der Hals (29) und Kopf (34) durch einen zerbrechlichen Bereich (48) des Kolbens (28) verbunden sind.

3. Die Spritze (20) gemäß Anspruch 2, wobei der zerbrechliche Bereich (48) einen gabelkopf-förmigen Vorsprung (49), der mit dem Hals (29) verbunden ist, und einen Stift (54), der mit dem Kopf (34) verbunden ist, einschliesst.

4. Die Spritze (20) gemäß Anspruch 3, wobei der zerbrechliche Bereich (48) vier Vorsprünge (51, 53) einschliesst, die den Stift (54) mit dem gabelkopf-förmigen Teil (49) trennbar verbinden.

5. Die Spritze (20) gemäß irgendeinem der vorhergehenden Ansprüche, wobei die erste Verbindungsstruktur (74) einen oder mehrere Zinken einschließt, die auf der inneren Wand des Zylinders (22) ausgeformt sind, und die zweite Verbindungsstruktur (46) eine Schulter einschließt, die auf dem Kopf ausgeformt ist und die sich mit einem oder mehreren der Zinken verbinden kann.

6. Die Spritze (20) gemäß irgendeinem der vorhergehenden Ansprüche, wobei der Kopf (34) eine Kolbendichtung (40) einschließt, die so gestaltet ist, dass sie dicht an einer inneren Wand des Zylinders (22) anliegt, wobei die zweite Verbindungsstruktur (46) an einer Vorderseite der Kolbendichtung (40) positioniert ist.

7. Die Spritze (20) gemäß irgendeinem der vorhergehenden Ansprüche, wobei die bestimmte in Längsrichtung gerichtete Kraft, die benötigt wird, um den Hals (29) vom Kopf (34) zu trennen, zwischen etwa 5N und 15N beträgt.

8. Die Spritze (20) gemäß irgendeinem der vorhergehenden Ansprüche, wobei die Fangposition erreicht ist, sobald der Kopf (34) mindestens 95 % des Hubs durchmessen hat.

9. Die Spritze (20) gemäß Anspruch 1, wobei das erste Rastteil (71) einen Vorsprung einschließt, der von der inneren Wand des Zylinders (22) in einen Hohlraum (23) des Zylinders (22) ragt und dadurch den Transversalquerschnitt des Hohlraums (23) lokal vermindert.

10. Die Spritze (20) gemäß Anspruch 9, wobei das zweite Rastteil (64) eine Protuberanz einschließt, die auf dem Hals (29) ausgeformt ist.

11. Die Spritze (20) gemäß Anspruch 10, wobei die Protuberanz durch Verbindung mit dem Vorsprung verformbar ist.

12. Die Spritze (20) gemäß Anspruch 10 oder 11, wobei die Protuberanz zwei Rippen (64a, 64b) einschließt, die von gegenüberliegenden Seiten des Halses (29) hervorragen.

13. Die Spritze (20) gemäß Anspruch 12, wobei jede Rippe (64a, 64b) einen zentralen Teil einschließt, der mit dem Hals (29) an ersten und zweiten Enden verbunden ist, so dass eine Öffnung zwischen dem zentralen Teil und dem Hals (29) definiert wird.

14. Die Spritze (20) gemäß irgendeinem der vorhergehenden Ansprüche, wobei die erste Verbindungsstruktur integral mit dem Zylinder (22) ausgeformt ist.

15. Die Spritze (20) gemäß irgendeinem der vorhergehenden Ansprüche, wobei der Zylinder (22) einen Hohlraum (23) hat, der sich zwischen dem vorderen Ende (26) des Zylinders (22) und dem hinteren Ende (30) des Zylinders (22) erstreckt, wobei der Hohlraum (23) einen vorderen Hohlraumteil (23a) und einen hinteren Hohlraumteil (23b) umfasst, die durch eine Begrenzung getrennt sind, welche durch die erste Verbindungsstruktur (74) definiert ist, und wobei die Einwegspritze (20) zusätzlich umfasst:
eine Nadelbefestigung (66), die innerhalb des vorderen Hohlraumteils (23a) angebracht ist, wobei die Nadelspitze (24) auf der Nadelbefestigung (66) befestigt ist, und wobei der Kolben (28) in den hintere Hohlraumteil (23b) eingeführt werden kann, wobei der Kolben (28) durch einen Längshub in der Längsrichtung bis zu einer Fangposition an einem Ende des Hubs verschiebbar ist, in welcher sich die erste Verbindungsstruktur (74) mit der zweiten Verbindungsstruktur (46) verbindet und dadurch den Kolben (28) im hinteren Hohlraumteil (23b) sichert.

16. Die Spritze (20) gemäß Anspruch 15, wobei die Nadelbefestigung (66) eine sich radial erstreckende Lippe (68) einschließt, die in einer entsprechenden ringförmige Vertiefung (70) innerhalb des vorderen Hohlraumteils (23a) gehalten wird.

17. Verfahren zur Ausformung eines Zylinders (22) einer Spritze (20), wobei das Verfahren die Schritte einschließt:
Positionieren eines Plastikrohlings in einer Matrize, die eine Längsachse hat, welche sich in eine Längsrichtung erstreckt, und
Pressen des Rohlings von einer ersten Seite entlang der Längsachse, wodurch ein vorderer Hohlraumteil (23a) zur Aufnahme einer Nadelbefestigung (66) ausgeformt wird,
Pressen des Rohlings von einer zweiten gegenüberliegenden Seite, wodurch ein hinterer Hohlraumteil (23b) zur Aufnahme eines Kolbens (28) ausgeformt wird,
wobei das Pressen von der ersten Seite und das Pressen von der zweiten Seite auch eine erste Verbindungsstruktur (74) zur Verbindung eines vorderen Kopfteils (34) des Kolbens (28) schafft, wobei die erste Verbindungsstruktur (74) durch eine Begrenzung definiert wird, die den vorderen Hohlraumteil (23a) und den hinteren Hohlraumteil (23b) trennt;
**dadurch gekennzeichnet, dass** ein erstes Rastteil (71) auf der inneren Wand des Zylinders (22) ausgeformt ist, das so gestaltet ist, dass es Teil einer Rasteinrichtung ist, die mit dem Zylinder (22) und dem Kolben (28) verbunden ist, wobei die Rasteinrichtung so gestaltet ist, dass sie die Verschiebung des Kolbens (28) in der Längsrichtung verhindert, sobald sich der Kolben (28) einer Fangposition nähert, und dadurch, dass das erste Rastteil (71) so gestaltet ist, dass es sich mit einem zweiten Rastteil (64), das auf dem Hals (29) des Kolbens (28) ausgeformt ist, zu verbindet, sobald sich der Kolben (28) der Fangposition nähert.

18. Das Verfahren gemäß Anspruch 17, wobei der Schritt der Ausformung der ersten Verbindungsstruktur (74) die Ausformung von einem oder mehreren Zinken einschließt.

## Revendications

1. Seringue à usage unique (20) comportant :
un cylindre creux (22) ayant un axe longitudinal s'étendant dans une direction longitudinale et incluant une première formation d'engagement (74) formée sur une paroi interne dudit cylindre (22),
une pointe d'aiguille (24) fixe par rapport audit cylindre (22), ladite pointe d'aiguille (24) étant en communication fluidique avec ledit cylindre (22), et
un piston (28) pouvant être inséré à l'intérieur dudit cylindre (22), ledit piston (28) ayant une tige (29) et une tête (34) reliée à ladite tige (29) de manière séparable, ladite tête (34) incluant une seconde formation d'engagement (46) située à une extrémité avant de ladite tête (34),
ledit piston (28) pouvant être déplacé pendant une course longitudinale dans ladite direction longitudinale jusqu'à une position captive à une fin de ladite course dans laquelle ladite première formation d'engagement (74) s'engage dans ladite seconde formation d'engagement (46), en bloquant ainsi le piston (28) à l'intérieur du cylindre (22),
dans laquelle, dans la position captive, la tête (34) est bloquée en empêchant ainsi un mouvement de la tête (34) pendant ladite course, et ladite tige (29) est adaptée pour se séparer de la tête (34) si une tentative est faite pour déplacer longitudinalement la tige (29) dans une direction d'insertion ou de rétraction de la course, par application d'une force longitudinale prédéterminée, de telle sorte que ladite tête (34) reste bloquée à l'intérieur dudit cylindre (22),
**caractérisée par** un agencement d'arrêt associé audit cylindre (22) et audit piston (28), dans lequel ledit agencement d'arrêt est configuré pour empêcher un déplacement dudit piston (28) dans ladite direction longitudinale lorsque ledit piston (28) s'approche de ladite position captive et,
dans laquelle ledit agencement d'arrêt inclut une première partie d'arrêt (71) formée sur ladite paroi interne dudit cylindre (22) et une seconde partie d'arrêt (64) formée sur ladite tige (29), ladite première partie d'arrêt (71) étant adaptée pour venir en contact avec ladite seconde partie d'arrêt (64) lorsque ledit piston (28) s'approche de ladite position captive.

2. Seringue (20) selon la revendication 1, dans laquelle la tige (29) et la tige (34) sont reliées par une région cassante (48) dudit piston (28).

3. Seringue (20) selon la revendication 2, dans laquelle la région cassante (48) inclut une saillie en forme de chape (49) reliée à ladite tige (29) et un ergot (54) relié à ladite tête (34).

4. Seringue (20) selon la revendication 3, dans laquelle ladite région cassante (48) inclut quatre tétons (51, 53) qui relient l'ergot (54) à ladite partie en forme de chape (49) de manière cassante.

5. Seringue (20) selon l'une quelconque des revendications précédentes, dans laquelle la première formation d'engagement (74) inclut une ou plusieurs dents formées sur une paroi interne dudit cylindre (22), et ladite seconde formation d'engagement (46) inclut un épaulement formé sur ladite tête et pouvant venir en contact avec ladite ou lesdites dents.

6. Seringue (20) selon l'une quelconque des revendications précédentes, dans laquelle ladite tête (34) inclut un joint de piston (40) adapté pour venir en contact de manière étanche avec une paroi interne dudit cylindre (22), dans laquelle ladite seconde formation d'engagement (46) est située sur un côté avant dudit joint de piston (40).

7. Seringue (20) selon l'une quelconque des revendications précédentes, dans laquelle ladite force longitudinale prédéterminée requise pour séparer la tige (29) de la tête (34) est comprise entre 5 N et 15 N.

8. Seringue (20) selon l'une quelconque des revendications précédentes, dans laquelle ladite position captive est atteinte lorsque ladite tête (34) s'est déplacée pendant au moins 95 % de ladite course.

9. Seringue (20) selon la revendication 1, dans laquelle ladite première partie d'arrêt (71) inclut une saillie qui dépasse de ladite paroi interne dudit cylindre (22) dans un espace creux (23) dudit cylindre (22), en réduisant ainsi localement une section transversale dudit espace creux (23).

10. Seringue (20) selon la revendication 9, dans laquelle ladite seconde partie d'arrêt (64) inclut une protubérance formée sur ladite tige (29).

11. Seringue (20) selon la revendication 10, dans laquelle ladite protubérance est déformable lors d'un contact avec ladite saillie.

12. Seringue (20) selon la revendication 10 ou 11, dans laquelle ladite protubérance inclut deux nervures (64a, 64b) faisant saillie à partir de côtés opposés de ladite tige (29).

13. Seringue (20) selon la revendication 12, dans laquelle chaque nervure (64a, 64b) inclut une partie centrale reliée à la tige (29) à des première et seconde extrémités, de telle sorte qu'une ouverture est définie entre la partie centrale et ladite tige (29).

14. Seringue (20) selon l'une quelconque des revendications précédentes, dans laquelle ladite première formation d'engagement est formée d'un seul tenant avec ledit cylindre (22).

15. Seringue (20) selon l'une quelconque des revendications précédentes,
dans laquelle ledit cylindre (22) a un espace creux (23) s'étendant entre une extrémité avant (26) dudit cylindre (22) et une extrémité arrière (30) dudit cylindre (22), ledit espace creux (23) comportant une partie creuse avant (23a) et une partie creuse arrière (23b) séparées par une restriction définie par ladite première formation d'engagement (74), et dans laquelle ladite seringue à usage unique (20) comporte en outre
une monture d'aiguille (66) montée à l'intérieur de ladite partie creuse avant (23a), dans lequel la pointe d'aiguille (24) est montée sur ladite monture d'aiguille (66), et dans lequel le piston (28) peut être inséré à l'intérieur de ladite partie creuse arrière (23b), ledit piston (28) étant déplaçable pendant une course longitudinale dans ladite direction longitudinale jusqu'à une position captive à une fin de ladite course dans laquelle ladite première formation d'engagement (74) s'engage dans ladite seconde formation d'engagement (46), en bloquant ainsi le piston (28) à l'intérieur de la partie creuse arrière (23b).

16. Seringue (20) selon la revendication 15, dans laquelle ladite monture d'aiguille (66) inclut une lèvre s'étendant radialement (68) qui est reçue de manière captive à l'intérieur d'une rainure annulaire correspondante (70) formée à l'intérieur de ladite partie creuse avant (23a).

17. Procédé de formation d'un cylindre (22) d'une seringue (20), ledit procédé incluant les étapes consistant à :
positionner une ébauche de matière plastique à l'intérieur d'une filière ayant un axe longitudinal s'étendant dans une direction longitudinale, et
presser ladite ébauche à partir d'un premier côté le long dudit axe longitudinal, en formant ainsi une partie creuse avant (23a) pour la réception d'une monture d'aiguille (66),
presser ladite ébauche à partir d'un second côté opposé en formant ainsi une partie creuse arrière (23b) pour la réception d'un piston (28),
dans lequel ladite pression à partir dudit premier côté et ladite pression à partir dudit second côté forme également une première formation d'engagement (74) pour s'engager dans une partie avant d'une tête (34) du piston (28), ladite première formation d'engagement (74) étant définie par une restriction qui sépare ladite partie creuse avant (23a) et ladite partie creuse arrière (23b),
**caractérisé en ce qu'**une première partie d'arrêt (71) est formée sur la paroi interne dudit cylindre (22) qui est configurée pour être une partie de l'agencement d'arrêt associé auxdits cylindre (22) et piston (28), dans lequel ledit agencement d'arrêt est configuré pour empêcher un déplacement dudit piston (28) dans ladite direction longitudinale lorsque ledit piston (28) s'approche d'une position captive, et **en ce que** ladite première partie d'arrêt (71) est adaptée pour venir en contact avec une seconde partie d'arrêt (64) formée sur la tige (29) dudit piston (28) lorsque ledit piston (28) s'approche de ladite position captive.

18. Procédé selon la revendication 17, dans lequel ladite étape de formation de ladite première formation d'engagement (74) inclut la formation d'une ou plusieurs dents moulées.
